# EUROPEAN PATENT SPECIFICATION

(11) **EP 3 682 789 B1**
(45) Date of publication and mention of the grant of the patent: **27.08.2025**
(21) Application number: 19211820.6
(22) Date of filing: 27.11.2019
(51) Int. Cl.: A61B 1/00, A61B 1/015

(54) **ENDOSCOPE, ENDOSCOPE SYSTEM, METHOD OF DETERMINING LEAKAGE OF ENDOSCOPE, AND LEAKAGE DETERMINATION PROGRAM FOR ENDOSCOPE**
ENDOSKOP, ENDOSKOPSYSTEM, VERFAHREN ZUR LECKAGEDETEKTION EINES ENDOSKOPS UND LECKAGEDETEKTIONSPROGRAMM FÜR EIN ENDOSKOP
ENDOSCOPE, SYSTÈME D'ENDOSCOPE, PROCÉDÉ DE DÉTERMINATION DE FUITE DE L'ENDOSCOPE ET PROGRAMME DE DÉTERMINATION DE FUITE DE L'ENDOSCOPE

(30) Priority: 18.01.2019 JP 2019006994
(43) Date of publication of application: 22.07.2020
(73) Proprietor: FUJIFILM Corporation, Tokyo 106-8620 (JP)
(72) Inventor: IIKURA, Takayuki, Kanagawa, Kanagawa 258-8538 (JP)
(74) Representative: Klunker IP Patentanwälte PartG mbB

(56) References cited:
- WO-A1-2018/207517
- DE-A1- 102012 220 372
- US-A1- 2011 301 414
- US-A1- 2016 081 531
- US-B1- 6 412 334

## Description

### BACKGROUND OF THE INVENTION

### 1. Field of the Invention

The present invention relates to an endoscope, an endoscope system, a method of determining the leakage of an endoscope, and a recording medium storing a leakage determination program for an endoscope.

### 2. Description of the Related Art

Generally, an airtight structure is employed in an endoscope to protect built-in elements, such as an image pickup element and a signal cable, from water. In a state where an enclosed space formed in the endoscope communicates with the outside (also referred to as a leakage state), water penetrates into the enclosed space. For this reason, there is a possibility that the built-in elements may be affected. Accordingly, a method of determining whether or not leakage occurs in an endoscope is proposed.

JP1998-286223A (JP-H10-286223A) discloses an endoscope device comprising a pump and a control unit that are provided in a light source device or an image processing device to be connected to an endoscope. The pump applies pressure to the inside of the endoscope, and a control unit detects the leakage of the endoscope on the basis of a change in the internal pressure of the endoscope.

JP1997-285442A (JP-H09-285442A) discloses an airtightness check unit that is to be connected to an endoscope. The airtightness check unit includes a pump applying pressure to the inside of the endoscope and a pressure sensor detecting the internal pressure of the endoscope, and detects the leakage of the endoscope on the basis of a change in the internal pressure of the endoscope.

US 2016/0081531 Al discloses an endoscope leak inspection apparatus that includes an endoscope connecting section communicably connected to an inside of an endoscope, a gas feed section that communicates with the endoscope connecting section and feeds gas, and a measuring section that measures an internal pressure of the endoscope. An endoscope-information recognizing section recognizes serial numbers uniquely allocated to individual endoscopes, and a storing section stores the serial numbers and measurement results of the measuring section in association with each other. A threshold setting section sets a threshold on the basis of measurement results in past having the same serial number stored in the storing section, and a leak determining section compares the threshold and a measurement result by the measuring section and determines presence or absence of a leak of the endoscope.

DE102012220372A1 discloses an endoscope and a pressure test method therefor.

WO2018207517A1 discloses a wireless endoscope having a controller that measures, by a gas pressure sensor, a gas pressure value inside an endoscope main body and determines whether or not the acquired gas pressure value is equal to or larger than a predetermined value.

US2011/301414A1 discloses an endoscope sheath including a body and one or more sensors disposed in the body.

US6412334B1 discloses that leak detection system evaluates the integrity the endoscope having an internal passage. The leak detection system includes an interior chamber connected to the internal passage.

### SUMMARY OF THE INVENTION

Even though leakage occurs in the endoscope, it is difficult for a large difference to be generated between the internal pressure of the enclosed space obtained in a state where pressure is applied to the enclosed space of the endoscope and the internal pressure of the enclosed space obtained when some time has passed from that state in a case where a hole allowing the enclosed space to communicate with the outside is very small. That is, in a case where this hole is small, the internal pressure of the enclosed space of the endoscope is gradually lowered over a long period, such as several tens of hours or several days.

Each of methods disclosed in JP1998-286223A (JP-H10-286223A) and JP1997-285442A (JP-H09-285442A) is to apply pressure to the inside of the endoscope and to detect leakage on the basis of a change in the internal pressure of the endoscope at the time of use of the endoscope or the like. Accordingly, since a change in the internal pressure of the endoscope cannot be monitored over the above-mentioned long period, in a case where very little leakage occurs, the very little leakage cannot be detected.

The invention has been made in consideration of the above-mentioned circumstances, and an object of the invention is to provide an endoscope, an endoscope system, a method of determining the leakage of an endoscope, and a recording medium storing a leakage determination program for an endoscope that can detect the occurrence of leakage with high accuracy.

In one aspect of the invention there is provided an endoscope system according to claim 1 of the appended claims.

In another aspect, there is provided a method of determining the leakage of an endoscope, according to claim 7 of the appended claims.

In another aspect, there is provided a recording medium storing a leakage determination program for an endoscope, according to claim 8 of the appended claims.

According to the invention, it is possible to provide an endoscope, an endoscope system, a method of determining the leakage of an endoscope, and a recording medium storing a leakage determination program for an endoscope that can detect the occurrence of leakage with high accuracy.

### BRIEF DESCRIPTION OF THE DRAWINGS

Fig. 1 is a diagram showing the schematic configuration of an endoscope system 200 that is an endoscope system according to an embodiment of the invention.
Fig. 2 is a schematic diagram showing the internal configuration of an endoscope device 100 of the endoscope system 200 shown in Fig. 1.
Fig. 3 is a schematic diagram showing the internal configuration of a pressure device 8 and an endoscope 1 of the endoscope system 200 shown in Fig. 1.
Fig. 4 is a flowchart illustrating an operation for determining the leakage of the endoscope 1 of the endoscope system 200 shown in Fig. 1.
Fig. 5 is a schematic diagram showing a modification example of the endoscope 1 shown in Fig. 3.
Fig. 6 is a flowchart illustrating an operation for determining the leakage of an endoscope 1 of an endoscope system 200 including the endoscope 1 shown in Fig. 5.

### DESCRIPTION OF THE PREFERRED EMBODIMENTS

An embodiment of the invention will be described below with reference to the drawings.

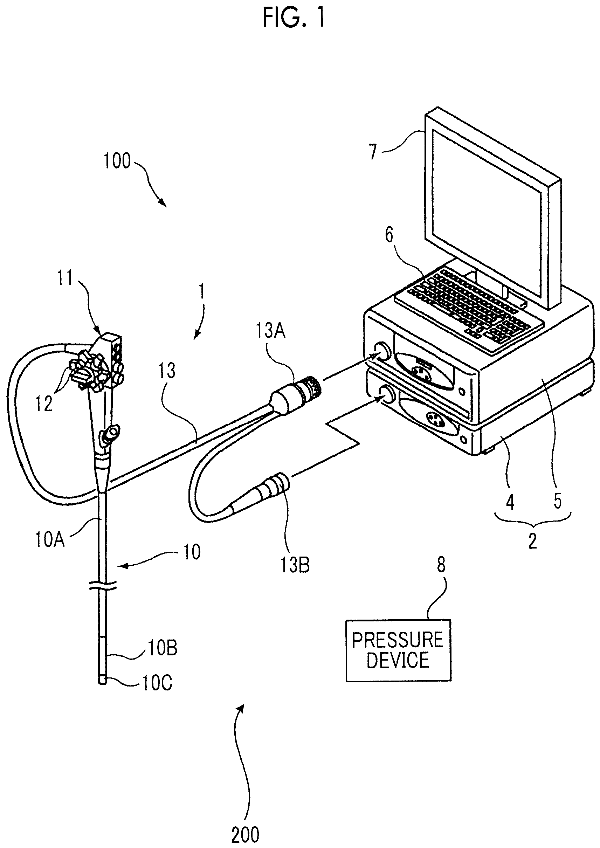

Fig. 1 is a diagram showing the schematic configuration of an endoscope system 200 that is an endoscope system according to an embodiment of the invention. As shown in Fig. 1, the endoscope system 200 comprises an endoscope device 100 and a pressure device 8.

The endoscope device 100 comprises an endoscope 1 and a main body 2 that includes a processor device 4 and a light source device 5 to which the endoscope 1 is to be connected. The main body 2 forms a device that can apply electric current to the endoscope 1.

A display unit 7 that displays a picked-up image and the like and an input unit 6 that is an interface used to input various kinds of information to the processor device 4 are connected to the processor device 4. The processor device 4 controls the endoscope 1, the light source device 5, and the display unit 7.

The endoscope 1 comprises: an insertion part 10 that is a tubular member extending in one direction and is to be inserted into a body cavity as an object to be observed; an operation part 11 that is connected to a proximal end portion of the insertion part 10 and is provided with operation members used to perform an observation mode-switching operation, an image pickup/recording operation, the operation of forceps, an air/water supply operation, a suction operation, the operation of an electric scalpel, and the like; angle knobs 12 that are provided on the operation part 11 to be adjacent to each other; and a universal cord 13 that includes connector parts 13A and 13B for attachably and detachably connecting the endoscope 1 to the light source device 5 and the processor device 4, respectively.

Although not shown in Fig. 1, a treatment tool passage into which a treatment tool, such as a pair of biopsy forceps or an electric scalpel, as a collection instrument for collecting biological tissue, such as cells or a polyp, is to be inserted is provided in the operation part 11 and the insertion part 10.

The insertion part 10 includes a soft portion 10A that has flexibility, a bendable portion 10B that is provided at the distal end of the soft portion 10A, and a hard distal end portion 10C that is provided at the distal end of the bendable portion 10B. The distal end portion 10C is a portion that is harder than the soft portion 10A and the bendable portion 10B.

The bendable portion 10B is adapted to be bendable by an operation for rotationally moving the angle knob 12. Since the bendable portion 10B can be bent at any angle in any direction according to a portion of an object or the like to be examined where the endoscope 1 is used, the bendable portion 10B can allow the distal end portion 10C to face in a desired direction.

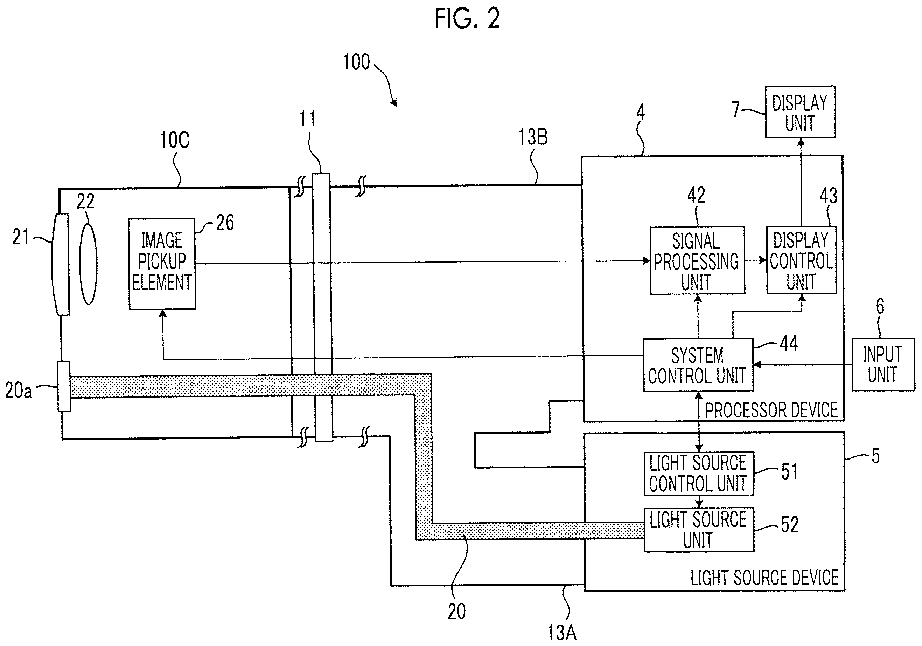

Fig. 2 is a schematic diagram showing the internal configuration of the endoscope device 100 of the endoscope system 200 shown in Fig. 1. As shown in Fig. 2, the light source device 5 comprises a light source control unit 51 and a light source unit 52.

The light source unit 52 is to generate illumination light that is used to illuminate a portion to be observed. Illumination light emitted from the light source unit 52 is incident on a light guide 20 built in the universal cord 13, and is applied to the portion to be observed through an illumination lens 20a that is provided in the distal end portion 10C of the insertion part 10.

A white light source emitting white light, a plurality of light sources that include a white light source and a light source emitting another color light (for example, a blue light source emitting blue light), or the like is used as the light source unit 52. Examples of a light-emitting element used as a light source in this specification include a laser diode (LD), a light-emitting diode (LED), and the like.

The light source control unit 51 is formed of various processors that execute programs to perform processing, and is connected to a system control unit 44 of the processor device 4. The light source control unit 51 controls the light source unit 52 on the basis of a command that is sent from the system control unit 44.

The distal end portion 10C of the endoscope 1 is provided with an image pickup optical system that includes an objective lens 21 and a lens group 22, an image pickup element 26 that picks up the image of a subject through the image pickup optical system, and a light guide 20 that guides illumination light emitted from the light source unit 52 to the illumination lens 20a.

The light guide 20 extends from the distal end portion 10C to the connector part 13A of the universal cord 13. A state where illumination light emitted from the light source unit 52 of the light source device 5 can be supplied to the light guide 20 is made in a state where the connector part 13A of the universal cord 13 is connected to the light source device 5. The light guide 20 is, specifically, an optical fiber bundle that is obtained in a case where a plurality of flexible optical fibers (for example, optical fibers made of plastic) are coated with a coating member in a state where the plurality of flexible optical fibers are bundled up, and transmits illumination light emitted from the light source unit 52 to the distal end portion 10C.

A charge coupled device (CCD) image sensor, a complementary metal oxide semiconductor (CMOS) image sensor, or the like is used as the image pickup element 26.

The image pickup element 26 includes a light-receiving surface on which a plurality of pixels are two-dimensionally arranged, converts an optical image, which is formed on the light-receiving surface by the image pickup optical system, into electrical signals (image pickup signals) at the respective pixels, and outputs the electrical signals. For example, an image pickup element on which color filters having primary colors, complementary colors, or the like are mounted is used as the image pickup element 26. The image pickup element 26 is electrically connected to the processor device 4 through a signal cable (not shown) that extends in the endoscope 1 from the distal end portion 10C to the connector part 13B.

In a case where a light source unit for dividing white light, which is emitted from a white light source, in a time-sharing manner by a plurality of color filters to generate illumination light is used as the light source unit 52, an image pickup element on which color filters are not mounted may be used as the image pickup element 26.

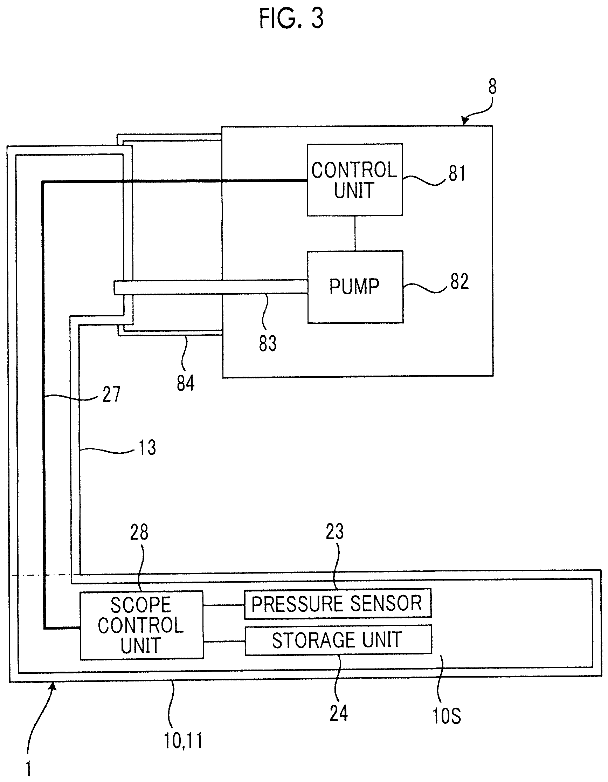

The inside of the endoscope 1 except for the above-mentioned treatment tool passage forms an enclosed space 10S (see Fig. 3), and built-in elements, such as the image pickup optical system, the image pickup element 26, and the signal cable and the like, are housed in the enclosed space 10S. Accordingly, the built-in elements are protected from water and the like.

The pressure device 8 is a device that is used to determine whether or not a leakage state where the enclosed space 10S of the endoscope 1 communicates with the outside is made.

The processor device 4 comprises a signal processing unit 42, a display control unit 43, and a system control unit 44.

The signal processing unit 42 receives and processes digital pixel signals, which are transmitted from the image pickup element 26, to generate picked-up image data. The picked-up image data, which is generated by the signal processing unit 42, is recorded in a recording medium, such as a hard disk drive or a flash memory (not shown).

The display control unit 43 causes the display unit 7 to display a picked-up image that is based on the picked-up image data generated by the signal processing unit 42.

The system control unit 44 controls the respective parts of the processor device 4, and sends a command to the endoscope 1 and the light source control unit 51 to generally control the entire endoscope device 100. The system control unit 44 performs the control of the image pickup element 26, the control of the light source unit 52 through the light source control unit 51, and the like.

The system control unit 44 includes various processors that execute programs to perform processing, a random access memory (RAM), and a read only memory (ROM).

Various processors of this specification include a central processing unit (CPU) that is a general-purpose processor executing programs to perform various kinds of processing, a programmable logic device (PLD) that is a processor of which circuit configuration can be changed after manufacture, such as a field programmable gate array (FPGA), a dedicated electrical circuit that is a processor having circuit configuration designed exclusively to perform specific processing, such as an application specific integrated circuit (ASIC), and the like. The structures of these various processors are more specifically electrical circuits where circuit elements, such as semiconductor elements, are combined.

The system control unit 44 may be formed of one of the various processors, or may be formed of a combination of two or more same kind or different kinds of processors (for example, a combination of a plurality of FPGAs or a combination of CPUs and FPGAs).

Fig. 3 is a schematic diagram showing the internal configuration of the pressure device 8 and the endoscope 1 of the endoscope system 200 shown in Fig. 1. A scope control unit 28, a pressure sensor 23, and a storage unit 24 are provided in the enclosed space 10S of the endoscope 1 at any portion, for example, the distal end portion 10C, the operation part 11, the connector part 13A, the connector part 13B, or the like.

The scope control unit 28 controls the endoscope 1 on the basis of the command of the system control unit 44 of the processor device 4 in a state where the endoscope 1 is connected to the processor device 4. The scope control unit 28 is formed of the above-mentioned various processors.

The pressure sensor 23 is to detect pressure inside the enclosed space 10S formed in the endoscope 1, and a capacitive sensor, a strain gauge type sensor, or the like is used as the pressure sensor 23. Information about the pressure detected by the pressure sensor 23 is transmitted to the scope control unit 28.

The storage unit 24 stores the pressure inside the enclosed space 10S, which is detected by the pressure sensor 23 at a first timing to be described later, (hereinafter, referred to as reference pressure P1) in association with detection time information t1 about the reference pressure P1. The storage unit 24 is formed of a storage medium, such as an electrically erasable programmable read-only memory (EEPROM) or a flash memory. The reference pressure P1 and the detection time information t1 are stored in the storage unit 24 by the control of the scope control unit 28. The reference pressure P1 forms a first pressure value. The detection time information t1 forms first detection time information.

The pressure device 8 comprises a control unit 81, a pump 82, an air supply tube 83, and a connector 84 and is operated by a power source (not shown).

The connector 84 is to be connected to the universal cord 13 of the endoscope 1. In a state where the connector 84 and the universal cord 13 are connected to each other, a state where the air supply tube 83 is inserted into the enclosed space 10S of the endoscope 1 is made and a state where the signal cable 27 of the endoscope 1 and the control unit 81 are electrically connected to each other is made.

The pump 82 sends air to the air supply tube 83 to apply pressure to the enclosed space 10S formed in the endoscope 1 connected to the connector 84. The pump 82 is controlled by the control unit 81. Since a portion of the endoscope 1 into which the air supply tube 83 is to be inserted has a check valve structure, the endoscope 1 is adapted so that air in the endoscope 1 does not leak from this portion in a state where the air supply tube 83 is detached.

The control unit 81 is electrically connected to the scope control unit 28, which is provided in the endoscope 1, through the signal cable 27.

In the endoscope system 200, pressure is applied to the enclosed space 10S of the endoscope 1 with respect to the atmospheric pressure by the pressure device 8 at a timing when the cleaned endoscope 1 is to be stored, or the like, and the reference pressure P1 and the detection time information t1, which are obtained in a state where pressure is applied, are stored in the storage unit 24. The endoscope 1 is stored until the time of next use in a state where the reference pressure P1 and the detection time information t1 are stored in the storage unit 24 in this way.

More specifically, the administrator of the endoscope 1 connects the universal cord 13 of the endoscope 1 to the connector 84 of the pressure device 8 first and starts the pressure device 8. Then, the administrator of the endoscope 1 operates an operation part (not shown) provided on the pressure device 8 to give an instruction to apply pressure to the enclosed space 10S of the endoscope 1. The control unit 81 of the pressure device 8 receiving this instruction causes the pump 82 to operate and instructs the scope control unit 28 to store the reference pressure P1 and the detection time information t1.

The scope control unit 28 receiving this instruction monitors the value of pressure detected by the pressure sensor 23 while pressure is applied to the enclosed space 10S by the pump 82; at a point of time when this pressure value becomes a peak, stores the pressure value of the peak in the storage unit 24 as the reference pressure P1; and stores the date and time at this point of time in the storage unit 24 as the detection time information t1 in association with the reference pressure P1. The point of time when this pressure value becomes a peak is the above-mentioned first timing. In this way, pressure inside the enclosed space 10S, which is obtained in a state where pressure is applied to the enclosed space 10S with respect to the atmospheric pressure, and information about the date and time at a point of time when the internal pressure of the enclosed space 10S becomes the pressure are stored in the storage unit 24 of the endoscope 1 in association with each other.

In the endoscope system 200, the scope control unit 28 of the endoscope 1 executes programs, which include a leakage (also refer to as "airtight leak") determination program, to perform processing for determining whether or not the leakage of the enclosed space 10S of the endoscope 1 occurs on the basis of the information stored in the storage unit 24 and information about the pressure of the enclosed space 10S detected by the pressure sensor 23. The scope control unit 28 forms a leakage determination unit. The details of this processing will be described below.

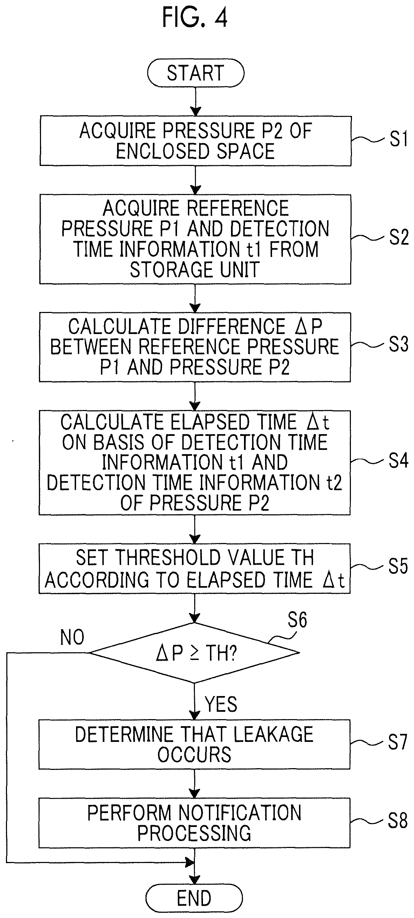

Fig. 4 is a flowchart illustrating an operation for determining the leakage of the endoscope 1 of the endoscope system 200 shown in Fig. 1. A case where the reference pressure P1 and the detection time information t1 have already been stored in the storage unit 24 of the endoscope 1 by the above-mentioned work will be described.

In a case where the connector parts 13A and 13B of the endoscope 1 are connected to the main body 2 and electric current is applied to the endoscope 1 from the main body 2, the scope control unit 28 acquires pressure P2 of the enclosed space 10S detected by the pressure sensor 23 and temporarily stores the pressure P2 in a built-in memory in association with detection time information t2 that represents the date and time of the detection of the pressure P2 (Step S1). The pressure P2 forms a second pressure value. The detection time information t2 forms second detection time information. Date and time, which are represented by the detection time information t2, form a second timing.

Then, the scope control unit 28 acquires the reference pressure P1 and the detection time information t1 from the storage unit 24 and temporarily stores the reference pressure P1 and the detection time information t1 in the built-in memory (Step S2).

After that, the scope control unit 28 calculates a difference ΔP between the reference pressure P1 and the pressure P2 (Step S3). In a case where leakage does not occur in the enclosed space 10S of the endoscope 1, this difference ΔP is reduced. On the other hand, in a case where leakage occurs in the enclosed space 10S of the endoscope 1, this difference ΔP is increased. Accordingly, leakage can be determined through the comparison of the magnitude of this difference ΔP and a threshold value TH to be described later.

Furthermore, the scope control unit 28 calculates elapsed time Δt, which has passed until a point of time (second timing) when the pressure P2 of the enclosed space 10S is detected from a point of time (first timing) when pressure inside the enclosed space 10S of the endoscope 1 becomes the reference pressure P1, on the basis of the detection time information t1 and the detection time information t2 (Step S4).

Then, the scope control unit 28 sets the threshold value TH, which is to be compared with the difference ΔP for the determination of leakage, according to the elapsed time Δt (Step S5).

Even in a case where leakage does not occur in the enclosed space 10S of the endoscope 1, air present in the enclosed space 10S gradually leaks to the outside due to the material of a member forming the exterior of the endoscope 1 or the like although the amount of air to leak is not so much. The amount of air to leak is larger at time (the elapsed time Δt) between the timing when the reference pressure P1 is detected and the timing when the pressure P2 is detected is longer.

In this embodiment, a plurality of values corresponding to the length of the elapsed time Δt are determined in advance as the threshold value TH to improve the accuracy of the determination of leakage. For example, a value TH1 corresponding to a case where the elapsed time Δt becomes a first value exceeding a predetermined value and a value TH2 corresponding to a case where the elapsed time Δt becomes a second value equal to or smaller than the predetermined value are determined as the threshold value TH, and the value TH1 is larger than the value TH2.

In a case where leakage does not occur, a value, which is larger than the upper limit of the magnitude of the difference ΔP assumed to be generated in a case where the elapsed time Δt becomes the first value, is set as the value TH1. In a case where leakage does not occur, a value, which is larger than the upper limit of the magnitude of the difference ΔP assumed to be generated in a case where the elapsed time Δt becomes the second value, is set as the value TH2. In Step S5, the scope control unit 28 sets the value TH1 as the threshold value TH in a case where the elapsed time Δt is the first value and sets the value TH2 as the threshold value TH in a case where the elapsed time Δt is the second value.

After Step S5, the scope control unit 28 determines whether or not the difference ΔP calculated in Step S3 is equal to or larger than the set threshold value TH (Step S6). In a case where the difference ΔP is equal to or larger than the threshold value TH (YES in Step S6), the scope control unit 28 determines that leakage occurs in the enclosed space 10S of the endoscope 1 (Step S7) and performs notification processing (Step S8).

For example, the scope control unit 28 performs notification processing for causing the display unit 7 to display a predetermined message (a warning message for displaying that leakage occurs) through the system control unit 44. The scope control unit 28 may cause the message to be output from a speaker (not shown), which is provided in the endoscope device 100, instead of causing the display unit 7 to display a message. Alternatively, the scope control unit 28 may send the message to an external electronic device, which is connected to the processor device 4, to notify the administrator of the endoscope 1 of necessity of repair.

In a case where the difference ΔP is smaller than the threshold value TH (NO in Step S6), the scope control unit 28 determines that leakage does not occur in the enclosed space 10S of the endoscope 1 and ends an operation for determining the leakage.

As described above, whether or not leakage occurs is determined in the endoscope system 200 on the basis of the magnitude of the difference ΔP between the pressure P2 of the enclosed space 10S at a timing when the determination of leakage is performed (in the above-mentioned example, a timing when the endoscope 1 is connected to the processor device 4 and electric current is applied to the endoscope 1) and the reference pressure P1 that is stored in the storage unit 24 in advance. For this reason, time between a timing when the reference pressure P1 is detected and a timing when the determination of leakage is performed (a timing when the pressure P2 is detected) can be set to long time corresponding to the storage period of the endoscope 1. As a result, since the difference ΔP is increased during this long time even though minute leakage occurs in the endoscope 1, the leakage can be detected. Accordingly, the determination of leakage can be performed with high accuracy.

Further, according to the endoscope system 200, since the threshold value TH is controlled according to the elapsed time Δt, a determination can be made in a consideration of a change in the internal pressure of the enclosed space 10S caused by a factor other than leakage. Accordingly, the determination of leakage can be performed with high accuracy.

Furthermore, according to the endoscope system 200, the scope control unit 28 of the endoscope 1 determines leakage. For this reason, since the main body 2 of the endoscope device 100 does not need to be significantly revamped, costs required to construct the system can be reduced. Moreover, since an additional function can be added to the existing endoscope device 100 by merely the interchange of the endoscope 1, versatility can be improved.

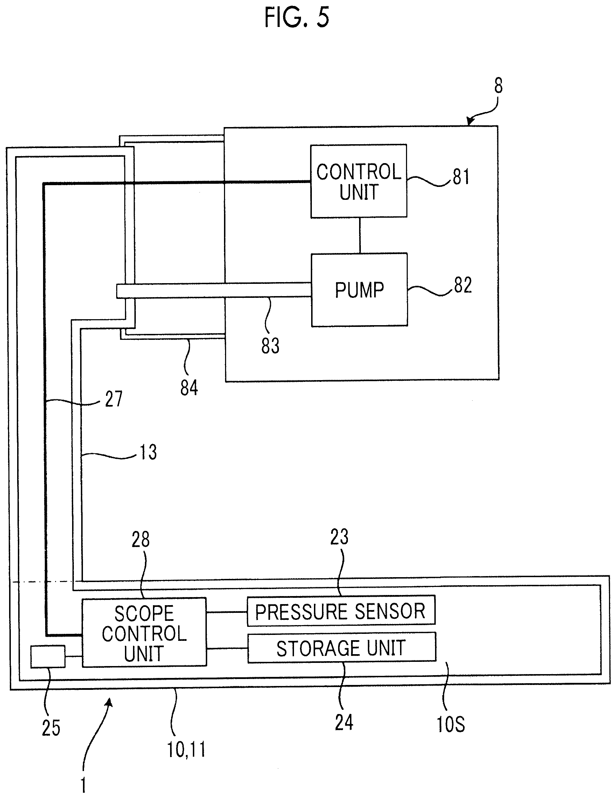

Fig. 5 is a schematic diagram showing a modification example of the endoscope 1 shown in Fig. 3. The endoscope 1 of the modification example shown in Fig. 5 has the same configuration as that of the endoscope 1 shown in Fig. 3 except that a temperature sensor 25 for detecting the temperature of the enclosed space 10S is added to the endoscope 1 of the modification example. The temperature sensor 25 transmits information about the detected temperature of the enclosed space 10S to the scope control unit 28.

The scope control unit 28 of the endoscope 1 shown in Fig. 5 is different from that of the endoscope 1 shown in Fig. 3 in that the scope control unit 28 of the endoscope 1 shown in Fig. 5 stores temperature T1 of the enclosed space 10S, which is detected by the temperature sensor 25 at a point of time when the reference pressure P1 is detected, in the storage unit 24 in association with the reference pressure P1 in a case where the scope control unit 28 stores the reference pressure P1 and the detection time information t1 in the storage unit 24. The temperature T1 forms first temperature.

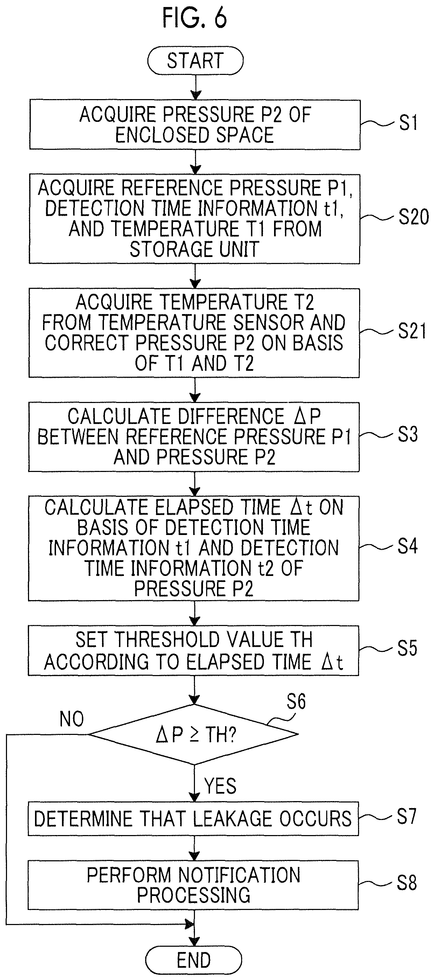

Fig. 6 is a flowchart illustrating an operation for determining the leakage of the endoscope 1 of the endoscope system 200 including the endoscope 1 shown in Fig. 5. The flowchart shown in Fig. 6 is the same as the flowchart shown in Fig. 4 except that Step S2 is replaced with Step S20 and Step S21. The same processing of Fig. 6 as that of Fig. 4 will be denoted by the same reference numeral as those of Fig. 4, and the description thereof will be omitted.

After Step S1, the scope control unit 28 acquires the reference pressure P1, the detection time information t1, and the temperature T1 from the storage unit 24, and temporarily stores the reference pressure P1, the detection time information t1, and the temperature T1 in a built-in memory (Step S20).

Then, the scope control unit 28 acquires temperature T2 of the enclosed space 10S, which is detected by the temperature sensor 25 at a point of time when the pressure P2 is detected, and corrects the pressure P2 acquired in Step S1 on the basis of the temperature T2 and the temperature T1 (Step S21). The temperature T2 forms second temperature.

In Step S3 and processing subsequent to Step S3 after Step S21, a value corrected in Step S21 is used as the pressure P2.

The internal pressure of the enclosed space 10S can be changed due to the temperature of the enclosed space 10S. For example, in a case where the temperature of the enclosed space 10S is high even though the internal pressure of the enclosed space 10S is constant, the internal pressure rises due to the thermal expansion of air present in the enclosed space 10S. In a case where the temperature of the enclosed space 10S is low, the internal pressure is lowered in reverse.

Accordingly, the scope control unit 28 performs correction for converting the value of the pressure P2, which is acquired in Step S1, into a value, which is obtained in a case where the temperature of the enclosed space 10S is the temperature T1, in Step S21 to improve the accuracy of the calculation of the difference ΔP. Specifically, the scope control unit 28 calculates a value, which is the product of the pressure P2 acquired in Step S1 and (T1/T2), as corrected pressure P2.

Since the difference ΔP is calculated as the amount of pressure changed to be obtained under the same temperature in this way, the determination of leakage can be performed with higher accuracy.

Processing for determining leakage shown in Fig. 4 or 6 may be performed in a case where the processor of the system control unit 44 of the processor device 4 connected to the endoscope 1 executes a leakage determination program. In this case, the processor of the system control unit 44 functions as a leakage determination unit. According to this configuration, the manufacturing cost of the endoscope 1 can be reduced.

Alternatively, the processing for determining leakage shown in Fig. 4 or 6 may be performed in a case where the control unit 81 of the pressure device 8 executes a leakage determination program. In this case, a flow where a user connects the endoscope 1 to the pressure device 8 and operates the operation part to cause the control unit 81 to start performing the processing for determining leakage immediately before the endoscope 1 is used, or the like is made. In this configuration, the control unit 81 of the pressure device 8 forms a leakage determination unit and the pressure device 8 forms a device that can apply electric current to the endoscope 1. According to this configuration, the manufacturing cost of the endoscope 1 can be reduced.

The scope control unit 28 of the endoscope 1 has performed control to store the reference pressure P1 and the detection time information t1 in the storage unit 24 in the above description, but is not limited thereto. For example, the control unit 81 of the pressure device 8 is adapted to be capable of having direct access to the pressure sensor 23 and the storage unit 24 through the signal cable 27. Further, the control unit 81 may be adapted to acquire information about pressure detected by the pressure sensor 23 and to store the reference pressure P1 and the detection time information t1 in the storage unit 24. According to this configuration, the manufacturing cost of the endoscope 1 can be reduced.

Even in the endoscope 1 shown in Fig. 5, likewise, the control unit 81 of the pressure device 8 may be adapted to store the reference pressure P1, the detection time information t1, and the temperature T1 in the storage unit 24.

The enclosed space 10S may be present even in the universal cord 13 in the examples shown in Figs. 3 and 5, but is not limited thereto. There is also a case where the enclosed space 10S of the endoscope 1 is present only in a portion closer to the distal end portion 10C than the universal cord 13. In this case, a portion into which the pump 82 may be to be inserted is provided near, for example, the operation part 11 of the endoscope 1 so that pressure can be applied to the enclosed space 10S.

Pressure, which is detected in a state where pressure is applied to the enclosed space 10S with respect to the atmospheric pressure by the pump 82, has been employed as the reference pressure P1 in the above description. However, pressure, which is detected in a state where pressure is reduced from the enclosed space 10S with respect to the atmospheric pressure by the pump 82, may be employed as the reference pressure P1. Even in this case, the difference ΔP is increased as the internal pressure of the enclosed space 10S becomes closer to the atmospheric pressure in a case where leakage occurs. For this reason, the determination of leakage can be performed on the basis of the magnitude of the difference ΔP.

Further, each of the detection time information t1 and the detection time information t2 has been information about date and time in the above description, but is not limited thereto. Each of the detection time information t1 and the detection time information t2 may be information about time that is elapsed from a point of time when the reference pressure P1 is detected.

### Explanation of References

100: endoscope device
200: endoscope system
1: endoscope
2: main body
20: light guide
20a: illumination lens
21: objective lens
22: lens group
23: pressure sensor
24: storage unit
25: temperature sensor
26: image pickup element
27: signal cable
28: scope control unit
4: processor device
42: signal processing unit
43: display control unit
44: system control unit
5: light source device
51: light source control unit
52: light source unit
6: input unit
7: display unit
8: pressure device
81: control unit
82: pump
83: air supply tube
84: connector
10: insertion part
10A: soft portion
10B: bendable portion
10C: distal end portion
10S: enclosed space
11: operation part
12: angle knob
13: universal cord
13A, 13B: connector part

## Claims

1. An endoscope system (200) comprising:
an endoscope (1) including an enclosed space therein, the endoscope comprising:
a pressure sensor (23) configured to detect pressure inside the enclosed space (10S); and
a storage unit (24) that stores a first pressure value, which is detected by the pressure sensor (23) at a first timing in a state where pressure is applied to or removed from the enclosed space (10S) with respect to atmospheric pressure, in association with first detection time information about the first pressure value,
a leakage determination unit configured to determine leakage of the enclosed space (10S) on the basis of the first pressure value and the first detection time information stored in the storage unit (24) and a second pressure value that is detected by the pressure sensor (23) at a second timing after the first timing and second detection time information about the second pressure value;
wherein the leakage determination unit is configured to determine that the leakage of the enclosed space (10S) occurs in a case where a difference between the first pressure value and the second pressure value is equal to or larger than a threshold value, and configured to set the threshold value on the basis of elapsed time that has passed until the second timing from the first timing, based on the first detection time information and the second detection time information; and
wherein the endoscope (1) is configured to be connected to a main body of an endoscope device (100) that is capable of applying current to the endoscope (1) and the second timing is a timing when the endoscope (1) is connected to the main body.

2. The endoscope system according to claim 1, further comprising:
a temperature sensor (25) configured to detect a temperature of the enclosed space (10S),
wherein the storage unit (24) stores a first temperature of the enclosed space (10S) at the first timing, which is detected by the temperature sensor (25), in association with the first pressure value.

3. The endoscope system according to claim 2,
wherein the temperature sensor (25) is configured to detect a second temperature of the enclosed space (10S) at the second timing; and
wherein the leakage determination unit is configured to correct the second pressure value on the basis of the second temperature and the first temperature.

4. The endoscope system according to any of claims 1 to 3,
wherein the leakage determination unit is configured to set the threshold value, which is to be set in a case where the elapsed time has a first value, to a value larger than the threshold value which is to be set in a case where the elapsed time has a second value smaller than the first value.

5. The endoscope system according to any one of claims 1 to 4,
wherein the leakage determination unit is provided in the endoscope (1).

6. The endoscope system according to any one of claims 1 to 5,
wherein the leakage determination unit is provided in a main body of an endoscope device (100) that is connected to the endoscope (1).

7. A method of determining leakage of an endoscope (1) including a pressure sensor (23) that detects pressure inside an enclosed space (10S) and a storage unit (24) that stores a first pressure value, which is detected by the pressure sensor (23) at a first timing in a state where pressure is applied to or removed from the enclosed space (10S) with respect to atmospheric pressure, in association with first detection time information about the first pressure value, wherein the endoscope (1) is configured to be connected to a main body of an endoscope device (100) that is capable of applying current to the endoscope (1), the method comprising:
determining (S6, S7), using a leakage determination unit, leakage of the enclosed space (10S) on the basis of the first pressure value and the first detection time information stored in the storage unit and a second pressure value that is detected by the pressure sensor (23) at a second timing after the first timing and second detection time information about the second pressure value;
wherein the leakage determination unit determines that the leakage of the enclosed space occurs in a case where a difference between the first pressure value and the second pressure value is equal to or larger than a threshold value, and sets the threshold value on the basis of elapsed time that has passed until the second timing from the first timing, based on the first detection time information and the second detection time information; and
wherein the second timing is a timing when the endoscope (1) is connected to the main body.

8. A recording medium storing a leakage determination program for an endoscope (1) including a pressure sensor (23) that detects pressure inside an enclosed space (10S) and a storage unit (24) that stores a first pressure value, which is detected by the pressure sensor (23) at a first timing in a state where pressure is applied to or removed from the enclosed space (10S) with respect to atmospheric pressure, in association with first detection time information about the first pressure value, wherein the endoscope (1) is configured to be connected to a main body of an endoscope device (100) that is capable of applying current to the endoscope (1), the leakage determination program causing a computer to perform
a step of determining (S6, S7), using a leakage determination unit, leakage of the enclosed space (10S) on the basis of the first pressure value and the first detection time information stored in the storage unit and a second pressure value that is detected by the pressure sensor (23) at a second timing after the first timing and second detection time information about the second pressure value;
wherein the leakage determination unit determines that the leakage of the enclosed space occurs in a case where a difference between the first pressure value and the second pressure value is equal to or larger than a threshold value, and sets the threshold value on the basis of elapsed time that has passed until the second timing from the first timing, based on the first detection time information and the second detection time information; and
wherein the second timing is a timing when the endoscope (1) is connected to the main body.

## Patentansprüche

1. Endoskopsystem (200), umfassend:
ein Endoskop (1), das einen geschlossenen Raum darin enthält, wobei das Endoskop umfasst:
einen Drucksensor (23), der so konfiguriert ist, dass er Druck innerhalb des geschlossenen Raums (10S) detektiert;
eine Speichereinheit (24), die einen ersten Druckwert, der von dem Drucksensor (23) zu einem ersten Zeitpunkt in einem Zustand, in dem Druck auf den geschlossenen Raum (10S) angewendet oder von diesem entfernt wird, in Bezug auf Atmosphärendruck detektiert wird, in Verbindung mit ersten Detektionszeitinformationen über den ersten Druckwert speichert,
eine Leckage-Bestimmungseinheit, die so konfiguriert ist, dass sie Leckage des geschlossenen Raums (10S) auf der Grundlage des ersten Druckwerts und der in der Speichereinheit (24) gespeicherten ersten Detektionszeitinformationen und eines zweiten Druckwerts, der von dem Drucksensor (23) zu einem zweiten Zeitpunkt nach dem ersten Zeitpunkt detektiert wird, und zweiter Detektionszeitinformationen über den zweiten Druckwert bestimmt;
wobei die Leckage-Bestimmungseinheit so konfiguriert ist, dass sie in einem Fall, in dem ein Unterschied zwischen dem ersten Druckwert und dem zweiten Druckwert gleich oder größer als ein Schwellenwert ist, bestimmt, dass die Leckage des geschlossenen Raums (10S) auftritt, und so konfiguriert ist, dass sie den Schwellenwert auf der Grundlage von verstrichener Zeit, die bis zu dem zweiten Zeitpunkt seit dem ersten Zeitpunkt vergangen ist, basierend auf den ersten Detektionszeitinformationen und den zweiten Detektionszeitinformationen einstellt; und
wobei das Endoskop (1) so konfiguriert ist, dass es mit einem Hauptkörper einer Endoskopvorrichtung (100), die in der Lage ist, Strom an das Endoskop (1) anzulegen, verbunden wird und der zweite Zeitpunkt ein Zeitpunkt ist, zu dem das Endoskop (1) mit dem Hauptkörper verbunden ist.

2. Endoskopsystem nach Anspruch 1, ferner umfassend:
einen Temperatursensor (25), der so konfiguriert ist, dass er eine Temperatur des geschlossenen Raums (10S) detektiert,
wobei die Speichereinheit (24) eine erste Temperatur des geschlossenen Raums (10S) zu dem ersten Zeitpunkt, die von dem Temperatursensor (25) detektiert wird, in Verbindung mit dem ersten Druckwert speichert.

3. Endoskopsystem nach Anspruch 2,
wobei der Temperatursensor (25) so konfiguriert ist, dass er eine zweite Temperatur des geschlossenen Raums (10S) zu dem zweiten Zeitpunkt detektiert; und
wobei die Leckage-Bestimmungseinheit so konfiguriert ist, dass sie den zweiten Druckwert auf der Grundlage der zweiten Temperatur und der ersten Temperatur korrigiert.

4. Endoskopsystem nach einem der Ansprüche 1 bis 3,
wobei die Leckage-Bestimmungseinheit so konfiguriert ist, dass sie den Schwellenwert, der in einem Fall, in dem die verstrichene Zeit einen ersten Wert aufweist, einzustellen ist, auf einen Wert, der größer als der Schwellenwert ist, der in einem Fall, in dem die verstrichene Zeit einen zweiten Wert, der kleiner als der erste Wert ist, aufweist, einzustellen ist, einstellt.

5. Endoskopsystem nach einem der Ansprüche 1 bis 4,
wobei die Leckage-Bestimmungseinheit in dem Endoskop (1) vorgesehen ist.

6. Endoskopsystem nach einem der Ansprüche 1 bis 5,
wobei die Leckage-Bestimmungseinheit in einem Hauptkörper einer Endoskopvorrichtung (100) vorgesehen ist, die mit dem Endoskop (1) verbunden ist.

7. Verfahren zum Bestimmen von Leckage eines Endoskops (1), das einen Drucksensor (23), der Druck innerhalb eines geschlossenen Raums (10S) detektiert, und eine Speichereinheit (24), die einen ersten Druckwert, der von dem Drucksensor (23) zu einem ersten Zeitpunkt in einem Zustand detektiert wird, in dem Druck auf den geschlossenen Raum (10S) angewendet oder von diesem entfernt wird, in Bezug auf Atmosphärendruck in Verbindung mit ersten Detektionszeitinformationen über den ersten Druckwert speichert, enthält, wobei das Endoskop (1) so konfiguriert ist, dass es mit einem Hauptkörper einer Endoskopvorrichtung (100), die in der Lage ist, Strom an das Endoskop (1) anzulegen, verbunden ist, wobei das Verfahren umfasst:
Bestimmen (S6, S7), unter Verwendung einer Leckage-Bestimmungseinheit, von Leckage des geschlossenen Raums (10S) auf der Grundlage des ersten Druckwerts und der in der Speichereinheit gespeicherten ersten Detektionszeitinformationen und eines zweiten Druckwerts, der von dem Drucksensor (23) zu einem zweiten Zeitpunkt nach dem ersten Zeitpunkt detektiert wird, und zweiter Detektionszeitinformationen über den zweiten Druckwert;
wobei die Leckage-Bestimmungseinheit in einem Fall, in dem ein Unterschied zwischen dem ersten Druckwert und dem zweiten Druckwert gleich oder größer als ein Schwellenwert ist, bestimmt, dass die Leckage des geschlossenen Raums auftritt, und den Schwellenwert auf der Grundlage von verstrichener Zeit, die bis zu dem zweiten Zeitpunkt seit dem ersten Zeitpunkt vergangen ist, basierend auf den ersten Detektionszeitinformationen und den zweiten Detektionszeitinformationen einstellt; und
wobei der zweite Zeitpunkt ein Zeitpunkt ist, zu dem das Endoskop (1) mit dem Hauptkörper verbunden ist.

8. Aufzeichnungsmedium, das ein Leckage-Bestimmungsprogramm für ein Endoskop (1) speichert, das einen Drucksensor (23), der Druck innerhalb eines geschlossenen Raums (10S) detektiert, und eine Speichereinheit (24), die einen ersten Druckwert, der von dem Drucksensor (23) zu einem ersten Zeitpunkt in einem Zustand detektiert wird, in dem Druck auf den geschlossenen Raum (10S) angewendet oder von diesem entfernt wird, in Bezug auf Atmosphärendruck in Verbindung mit ersten Detektionszeitinformationen über den ersten Druckwert speichert, enthält, wobei das Endoskop (1) so konfiguriert ist, dass es mit einem Hauptkörper einer Endoskopvorrichtung (100), die in der Lage ist, Strom an das Endoskop (1) anzulegen, verbunden ist, wobei das Leckage-Bestimmungsprogramm einen Computer veranlasst, auszuführen:
einen Schritt des Bestimmens (S6, S7), unter Verwendung einer Leckage-Bestimmungseinheit, von Leckage des geschlossenen Raums (10S) auf der Grundlage des ersten Druckwerts und der in der Speichereinheit gespeicherten ersten Detektionszeitinformationen und eines zweiten Druckwerts, der von dem Drucksensor (23) zu einem zweiten Zeitpunkt nach dem ersten Zeitpunkt detektiert wird, und zweiter Detektionszeitinformationen über den zweiten Druckwert;
wobei die Leckage-Bestimmungseinheit in einem Fall, in dem ein Unterschied zwischen dem ersten Druckwert und dem zweiten Druckwert gleich oder größer als ein Schwellenwert ist, bestimmt, dass die Leckage des geschlossenen Raums auftritt, und den Schwellenwert auf der Grundlage von verstrichener Zeit, die bis zu dem zweiten Zeitpunkt seit dem ersten Zeitpunkt vergangen ist, basierend auf den ersten Detektionszeitinformationen und den zweiten Detektionszeitinformationen einstellt; und
wobei der zweite Zeitpunkt ein Zeitpunkt ist, zu dem das Endoskop (1) mit dem Hauptkörper verbunden ist.

## Revendications

1. Système d'endoscope (200) comprenant :
un endoscope (1) incluant un espace clos en son sein, l'endoscope comprenant :
un capteur de pression (23) configuré pour détecter la pression à l'intérieur de l'espace clos (10S) ;
une unité de stockage (24) qui stocke une première valeur de pression, qui est détectée par le capteur de pression (23) à un premier instant dans un état où une pression est appliquée ou retirée de l'espace clos (10S) par rapport à la pression atmosphérique, en association avec des premières informations de temps de détection concernant la première valeur de pression,
une unité de détermination de fuite configurée pour déterminer une fuite de l'espace clos (10S) sur la base de la première valeur de pression et des premières informations de temps de détection stockées dans l'unité de stockage (24) et d'une deuxième valeur de pression qui est détectée par le capteur de pression (23) à un deuxième instant postérieur au premier instant et de deuxièmes informations de temps de détection concernant la deuxième valeur de pression ;
dans lequel l'unité de détermination de fuite est configurée pour déterminer que la fuite de l'espace clos (10S) se produit dans un cas où une différence entre la première valeur de pression et la deuxième valeur de pression est égale ou supérieure à une valeur seuil, et configurée pour définir la valeur seuil sur la base du temps écoulé qui s'est écoulé jusqu'au deuxième instant à partir du première instant, sur la base des premières informations de temps de détection et des deuxièmes informations de temps de détection ; et
dans lequel l'endoscope (1) est configuré pour être connecté à un corps principal d'un dispositif endoscopique (100) qui est capable d'appliquer un courant à l'endoscope (1) et le deuxième instant est un instant où l'endoscope (1) est connecté au corps principal.

2. Système d'endoscope selon la revendication 1, comprenant en outre :
un capteur de température (25) configuré pour détecter une température de l'espace clos (10S),
dans lequel l'unité de stockage (24) stocke une première température de l'espace clos (10S) au premier instant, qui est détectée par le capteur de température (25), en association avec la première valeur de pression.

3. Système d'endoscope selon la revendication 2,
dans lequel le capteur de température (25) est configuré pour détecter une deuxième température de l'espace clos (10S) au deuxième instant ; et
dans lequel l'unité de détermination de fuite est configurée pour corriger la deuxième valeur de pression sur la base de la deuxième température et de la première température.

4. Système d'endoscope selon l'une quelconque des revendications 1 à 3,
dans lequel l'unité de détermination de fuite est configurée pour définir la valeur seuil, qui doit être définie dans un cas où le temps écoulé a une première valeur, à une valeur supérieure à la valeur seuil qui doit être définie dans un cas où le temps é coulé a une deuxième valeur inférieure à la première valeur.

5. Système d'endoscope selon l'une quelconque des revendications 1 à 4,
dans lequel l'unité de détermination de fuite est disposée dans l'endoscope (1).

6. Système d'endoscope selon l'une quelconque des revendications 1 à 5,
dans lequel l'unité de détermination de fuite est disposée dans un corps principal d'un dispositif endoscopique (100) qui est connecté à l'endoscope (1).

7. Procédé de détermination de fuite d'un endoscope (1) incluant un capteur de pression (23) qui détecte la pression à l'intérieur d'un espace clos (10S) et une unité de stockage (24) qui stocke une première valeur de pression, qui est détectée par le capteur de pression (23) à un premier instant dans un état où une pression est appliquée ou retirée de l'espace clos (10S) par rapport à la pression atmosphérique, en association avec des premières informations de temps de détection concernant la première valeur de pression, où l'endoscope (1) est configuré pour être connecté à un corps principal d'un dispositif endoscopique (100) qui est capable d'appliquer un courant à l'endoscope (1), le procédé comprenant :
déterminer (S6, S7), à l'aide d'une unité de détermination de fuite, une fuite de l'espace clos (10S) sur la base de la première valeur de pression et des premières informations de temps de détection stockées dans l'unité de stockage et d'une deuxième valeur de pression qui est détectée par le capteur de pression (23) à un deuxième instant postérieur au premier instant et de deuxièmes informations de temps de détection concernant la deuxième valeur de pression ;
dans lequel l'unité de détermination de fuite détermine que la fuite de l'espace clos se produit dans un cas où une différence entre la première valeur de pression et la deuxième valeur de pression est égale ou supérieure à une valeur seuil, et définit la valeur seuil sur la base du temps écoulé qui s'est écoulé jusqu'au deuxième instant à partir du première instant, sur la base des premières informations de temps de détection et des deuxièmes informations de temps de détection ; et
dans lequel le deuxième instant est un instant où l'endoscope (1) est connecté au corps principal.

8. Support d'enregistrement stockant un programme de détermination de fuite pour un endoscope (1) incluant un capteur de pression (23) qui détecte la pression à l'intérieur d'un espace clos (10S) et une unité de stockage (24) qui stocke une première valeur de pression, qui est détectée par le capteur de pression (23) à un premier instant dans un état où une pression est appliquée ou retirée de l'espace clos (10S) par rapport à la pression atmosphérique, en association avec des premières informations de temps de détection concernant la première valeur de pression, où l'endoscope (1) est configuré pour être connecté à un corps principal d'un dispositif endoscopique (100) qui est capable d'appliquer un courant à l'endoscope (1), le programme de détermination de fuite amenant un ordinateur à effectuer
une étape consistant à déterminer (S6, S7), à l'aide d'une unité de détermination de fuite, une fuite de l'espace clos (10S) sur la base de la première valeur de pression et des premières informations de temps de détection stockées dans l'unité de stockage et d'une deuxième valeur de pression qui est détectée par le capteur de pression (23) à un deuxième instant postérieur au premier instant et de deuxièmes informations de temps de détection concernant la deuxième valeur de pression ;
dans lequel l'unité de détermination de fuite détermine que la fuite de l'espace clos se produit dans un cas où une différence entre la première valeur de pression et la deuxième valeur de pression est égale ou supérieure à une valeur seuil, et définit la valeur seuil sur la base du temps écoulé qui s'est écoulé jusqu'au deuxième instant à partir du première instant, sur la base des premières informations de temps de détection et des deuxièmes informations de temps de détection ; et
dans lequel le deuxième instant est un instant où l'endoscope (1) est connecté au corps principal.
